(19) Europäisches Patentamt
European Patent Office
Office européen des brevets

(11) Veröffentlichungsnummer: **0 023 335**
**A1**

(12) **EUROPÄISCHE PATENTANMELDUNG**

(21) Anmeldenummer: **80104280.5**

(22) Anmeldetag: **21.07.80**

(51) Int. Cl.$^3$: **C 07 D 233/14**
C 07 D 233/08, C 23 F 11/14
C 23 F 11/16, A 01 N 43/50
D 06 M 13/46, C 11 D 1/62
A 61 K 7/08

(30) Priorität: **30.07.79 DE 2930849**

(43) Veröffentlichungstag der Anmeldung:
**04.02.81 Patentblatt 81/5**

(84) Benannte Vertragsstaaten:
**AT BE CH DE FR GB IT LI NL SE**

(71) Anmelder: **Henkel Kommanditgesellschaft auf Aktien**
**-Patentabteilung- Postfach 1100 Henkelstrasse 67**
**D-4000 Düsseldorf 1(DE)**

(72) Erfinder: **Ploog, Uwe Dr.**
**Haydenweg 6**
**D-5657 Haan(DE)**

(72) Erfinder: **Uphues, Günter**
**Himmelgeister Strasse 143**
**D-4000 Düsseldorf(DE)**

(72) Erfinder: **Petzold, Manfred**
**Am Falder 93**
**D-4000 Düsseldorf 13(DE)**

(54) **Neue N-Hydroxyalkylimidazolinderivate, deren Herstellung und Verwendung.**

(57) Gegenstand der Anmeldung sind neue N-Hydroxyalkylimidazolinderivate der Formel

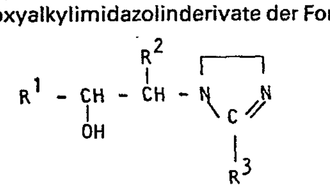

(1)

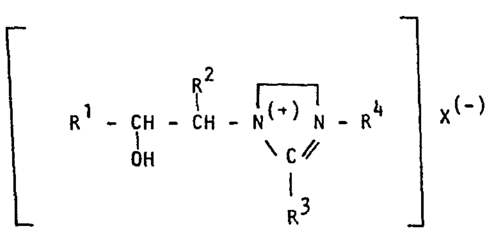

(11)

worin bedeuten:

$R^1$, $R^2$ = H und/oder unverzweigter $C_1$-$C_{20}$-Alkylrest, wobei die Summe der C-Atome in $R^1$ und $R^2$ 8 - 20 ist.

$R^3$ = H oder ein $C_1$-$C_{23}$-Alkylrest oder ein Phenylrest oder ein Rest $R^5$-A,

wobei $R^5$ ein um die Carboxylgruppen verminderter Rest einer aliphatischen oder aromatischen $C_3$-$C_{12}$-Dicarbonsäure,

A ein um $R^3$ verminderter Hydroxyalkylimidazolinrest ist,

$R^4$ = $C_1$-$C_{20}$-Alkyl oder Benzyl oder Propylsulfonat,

$X^{(-)}$ = beliebiges Anion einer organischen oder anorganischen Säure, insbesondere $Cl^{(-)}$, $X^{(-)}$ entfällt, wenn $R^4$ = Propylsulfonat. Weitere Gegenstände der Anmeldung sind die Herstellung der neuen Produkte durch Umsetzung entsprechender N-Hydroxyalkylethylendiamine mit einer Mono- oder Dicarbonsäure bei 120 - 150°C unter Abdestillieren des Reaktionswassers und gegebenenfalls weiterer Umsetzung mit einem Quatenierungsmittel, sowie deren Verwendung als Korrosionsschutzmittel, mikrobizide Mittel, Körper- und Haarwaschmittel oder als Textilavivage- und Textilweichmachungsmittel.

EP 0 023 335 A1

HENKEL KGaA
ZR-FE/Patente
Dr. Bz/ sü

P a t e n t a n m e l d u n g

D 5977 EP

Neue N-Hydroxyalkylimidazolinderivate, deren Herstellung und Verwendung

---

Die Erfindung betrifft neue N-Hydroxyalkylimidazoline und die davon abgeleiteten quaternären Imidazoliniumsalze, sowie deren Herstellung und Verwendung als mikrobizide Mittel, Korrosionsschutzmittel, Tenside und Textilavivagemittel.

Die neuen N-Hydroxyalkylimidazolinderivate besitzen die allgemeine Formel

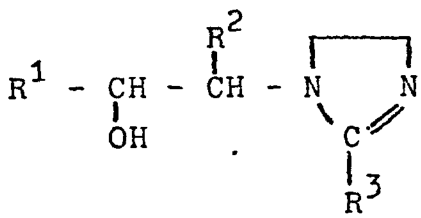

(I)

oder

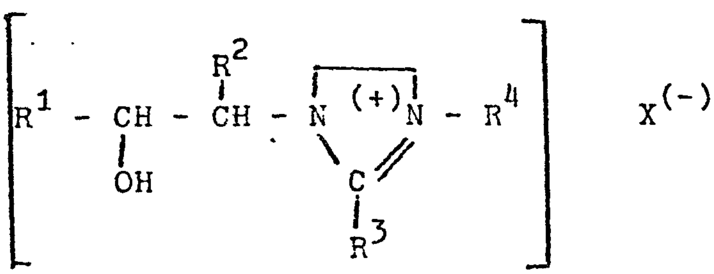

(II)

mit der im Patentanspruch 1 angegebenen Bedeutung für $R^1$, $R^2$, $R^3$, $R^4$ und $X^{(-)}$.

Zur Herstellung der Verbindungen geht man von N-Hydroxyalkylethylendiaminen der Formel

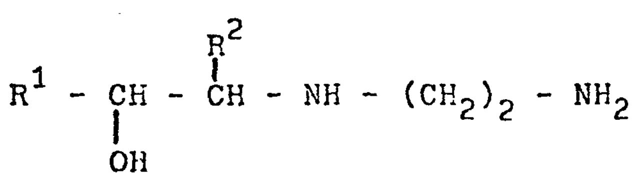

(III)

mit der im Patentanspruch 1 angegebenen Bedeutung für $R^1$ und $R^2$ aus. Verbindungen der Formel (III) werden erhalten durch Umsetzung von innen- oder vorzugsweise endständigen epoxidierten $C_{1o}$ - $C_{22}$-Olefinen mit Ethylendiamin. Ihre Herstellung ist beschrieben in DE-OS 25 2o 267 und DE-OS 25 47 987.

Die Verbindungen der Formel (III) werden durch weitere Reaktion mit annähernd äquivalenten Mengen einer Mono- oder Dicarbonsäure der Formel $R^3$-COOH oder HOOC-$R^5$-COOH, worin $R^3$ und $R^5$ die im Patentanspruch 1 angegebene Bedeutung haben, zu den N-Hydroxyalkylimidazolinen der Formel (I) umgesetzt. Geeignete Monocarbonsäuren sind lineare oder verzweigte, vorzugsweise gesättigte aliphatische $C_1$-$C_{24}$-Carbonsäuren, z. B. Ameisensäure, Essigsäure, Caprylsäure, Caprinsäure, Isopalmitinsäure, Stearinsäure. Es kommen jedoch auch aromatische Carbonsäuren, wie Benzoesäure oder Salicylsäure in Betracht. Bei Verwendung von aliphatischen oder aromatischen $C_3$-$C_{12}$-Dicarbonsäuren, wie Adipinsäure oder Terephthalsäure, entstehen durch Reaktion beider Carboxylgruppen symmetrische Bis-N-Hydroxyalkylimidazoline.

Die Umsetzung wird bei erhöhten Temperaturen von etwa 12o - 25o° C und vorzugsweise vermindertem Druck von 1o - 25o mbar unter Abdestillieren von 2 Mol Reaktionswasser pro Imidazolinring-Äquivalent durchgeführt. Der Zeitbedarf richtet sich nach Art und Kettenlänge der eingesetzten Carbonsäure, der Größe des Ansatzes, Temperatur, Druck u. dgl.. Im allgemeinen ist eine Reaktionszeit von 1 - 1o Stunden erforderlich. Durch die Verwendung eines Schleppmittels für die Abtrennung des Wassers, z. B. Xylol, Toluol oder Testbenzin kann die Reaktionszeit verkürzt werden. Die Reaktionsbedingungen entsprechen den üblichen Bedingungen für die Herstellung von Imidazolinen.

/3

Die N-Hydroxyalkylimidazoline können durch Zugabe äquivalenter Mengen einer organischen oder anorganischen Säure, z. B. Essigsäure, Salzsäure, Schwefelsäure oder Phosphorsäure, in die wasserlöslichen oder wasserdispergierbaren Salze übergeführt werden.

Durch Quaternierung der N-Hydroxyalkylimidazoline der Formel (I) kommt man zu den Imidazoliniumsalzen der Formel (II). Die Quaternierung erfolgt nach an sich bekannten Methoden durch Umsetzung mit Quaternierungsmitteln, wie Halogenkohlenwasserstoffen, z. B. Methylchlorid oder Benzylchlorid, oder durch alkylierende Substanzen wie Dimethylsulfat, Trimethylphosphat oder Propansulton. In letzterem Falle werden die entsprechenden Betaine erhalten.

Die Umsetzung wird ohne Lösungsmittel, oder, falls die Viskosität des Reaktionsgemisches zu hoch ist unter Verwendung geeigneter Verdünnungsmittel, z. B. Methanol oder Isopropanol bei Methylchlorid und Benzylchlorid; Dioxan oder dgl. bei Verwendung von Dimethylsulfat, gegebenenfalls unter Druck bis 5o bar, bei Temperaturen von 6o - 1oo$^{o}$ C durchgeführt. Nach Abdestillieren erhält man das wasserlösliche oder wasserdispergierbare quaternäre Imidazoliniumsalz.

Die neuen N-Hydroxyalkylimidazoline der Formel (I) weisen für sich oder in Form ihrer Salze eine hohe antimikrobielle und antikorrosive Wirksamkeit auf. Sie stellen gleichzeitig wertvolle Ausgangsstoffe für die Herstellung von Wasch- und Reinigungsmitteln, Textilavivagemitteln und Biociden dar, indem sie durch Quaternierung in die Imidazoliniumsalze der Formel (II) umgewandelt werden können. Letztere sind kationische Tenside, die neben einer hohen antimikrobiellen Wirkung eine ausgezeichnete Haut- und Schleimhautverträglichkeit besitzen,

weshalb sie für Körper- und Haarwaschmittel besonders geeignet sind. Ihre hohe Substantivität gegenüber festen Substratoberflächen macht sie zu besonders wirksamen Textilavivage- und -weichmachungsmitteln, die auf Synthesetextilmaterial eine hohe antistatische Wirksamkeitbesitzen und insbesondere Zellulosetextilien eine hohe Weichheit und Flauschigkeit bei guter Wiederbenetzbarkeit verleihen. Konventionelle kationische Avivagemittel führen demgegenüber zu einer mehr oder weniger ausgeprägten Hydrophobierung von Zellulosematerial, was sich insbesondere bei Handtüchern, Badetüchern und sonstigen Frotteegeweben beim Gebrauch störend bemerkbar macht.

Die Herstellung der in der nachfolgenden Tabelle 1 enthaltenen N-Hydroxyalkylimidazoline der Formel (I) erfolgt nach folgender allgemeiner Vorschrift:

In einer Apparatur, bestehend aus Dreihalskolben mit Rührung, Stickstoffspülung, Thermometer und Destillationsaufsatz sowie elektrischer Heizung wird 1 Äquivalent eines Diamins der Formel (III) vorgelegt und bei 70 - 90° C mit 1 Äquivalent der gewünschten Carbonsäure versetzt. Man erhitzt bei Normaldruck in 2 - 3 Stunden auf 200 - 220° C, während man den Druck allmählich auf etwa 250 - 25 mbar erniedrigt. Die Reaktion ist beendet, wenn 2 Äquivalente Wasser abgeschieden sind. Sofern das Diamin und/oder die Carbonsäure langkettige Alkylreste enthalten, empfiehlt sich die Anwendung eines Schleppmittels, z. B. Xylol, zum Abdestillieren des Wassers.

/5

**T a b e l l e 1**

N-Hydroxyalkylimidazoline

| Lfd. Nr. | Ausgangs-Olefin | Carbonsäure | $R^1$ | $R^2$ | $R^3$ | Äquivalenzverhältnis $N_{KJ}$ | $N_{titr}$ | Imidaz. | Fp °C |
|---|---|---|---|---|---|---|---|---|---|
| 1 | α-C 10 | Caprinsäure | $C_8H_{17}$ | H | $C_9H_{19}$ | 2,00 | 0,98 | 0,96 | wachs. |
| 2 | α-C 12 | Essigsäure | $C_{10}H_{21}$ | H | $CH_3$ | 2,00 | 0,99 | 1,13 | 73-74 |
| 3 | α-C 12 | Caprylsäure | $C_{10}H_{21}$ | H | $C_7H_{15}$ | 2,00 | 0,99 | 1,06 | flüssig |
| 4 | α-C 12 | Caprinsäure | $C_{10}H_{21}$ | H | $C_9H_{19}$ | 2,00 | 1,00 | 0,96 | 51-53 |
| 5 | α-C12/14 | Ameisensäure | $C_{10}-C_{12}$ | H | H | 2,00 | 1.01 | 1,03 | 58-59 |
| 6 | α-C12/14 | Essigsäure | $C_{10}-C_{12}$ | H | $CH_3$ | 2,00 | 0,95 | 1,03 | 60-61 |
| 7 | α-C12/14 | Stearinsäure | $C_{10}-C_{12}$ | H | $C_{15}-C_{17}$ | 2,00 | 1,02 | 0,98 | 49-50 |
| 8 | α-C16/18 | Essigsäure | $C_{14}-C_{16}$ | H | $CH_3$ | 2,00 | 1,01 | 1,01 | 70-71 |
| 9 | α-C16/18 | Stearinsäure | $C_{14}-C_{16}$ | H | $C_{15}-C_{17}$ | 2,00 | 1,13 | 1,11 | 65 |
| 10 | i-C11/14 | Essigsäure | $\sum C_9 - C_{13}$ | | $CH_3$ | 2,00 | 1,05 | 0,79 | flüssig |
| 11 | α-C12/14 | Isopalmitinsäure | $C_{10}-C_{12}$ | H | $C_{15}H_{31}$ | 2,00 | 1,06 | 0,66 | wachs. |
| 12 | α-C12/14 | Benzoesäure | $C_{10}-C_{12}$ | H | $C_6H_5$ | 2,00 | 1,03 | + | flüssig |
| 13 | α-C12/14 | Adipinsäure | $C_{10}-C_{12}$ | H | $C_4H_8$ | 2,00 | 1,04 | 0,80 | wachs. |

Erläuterungen: $N_{KJ}$ = Stickstoff nach Kjeldahl
$N_{titr}$ = potentiometrisch mit Perchlorsäure ermittelt
Imidaz. = Imidazolingehalt ermittelt über UV-Absorption bei 230 nm
+ = spektroskopisch nicht zu ermitteln
wachs. = wachsartig

/6

Beispiele für die Herstellung von N-Hydroxyalkylimidazolinen der Formel (I) und deren Salze:

Imidazolin Nr. 2 (Tabelle 1)

250 g (1,0 Mol) N-(2-Hydroxydodecyl)-ethylendiamin, hergestellt aus α-C12-Epoxid und Ethylendiamin, wurden geschmolzen und in einer Rührapparatur mit angeschlossenem Liebigkühler unter Inertgas bei 90° C mit 63,0 g (1,05 Mol) Essigsäure versetzt. Die Temperatur erhöhte sich auf 130° C. Anschließend wurde das Reaktionsgemisch gleichmäßig innerhalb von 2 Stunden auf 200° C erhitzt. Dabei wurden, beginnend bei 145° C, 23 g Wasser abdestilliert. Unter Druckerniedrigung auf 250 mbar und einer allmählichen Temperatursteigerung auf 220° C wurde die Reaktion während 2 Stunden fortgesetzt. Es wurden noch weitere 15 g Destillat erhalten.

Imidazolin Nr. 9 (Tabelle 1)

In einer mit einem Wasserabscheider ausgerüsteten Rührapparatur wurden 77,5 g (0,25 Mol) N-(2-Hydroxy-C16/18-alkyl)-ethylendiamin, 67,8 g (0,25 Mol) technische Stearinsäure und 100 ml Xylol unter Rückfluß erhitzt. Nach einer Reaktionszeit von 3 Stunden bei 155 - 160° C waren 7,6 ml Wasser im Wasserabscheider abgetrennt. Danach wurden unter allmählicher Druckerniedrigung auf 25 mbar sowie langsamer Temperatursteigerung auf 220° C das Lösungsmittel und restliches Reaktionswasser abdestilliert.

Imidazolin Nr. 12 (Tabelle 1)

In einer Rührapparatur entsprechend Herstellungsbeispiel für Imidazolin Nr. 2 wurden 245,3 g (1,0 Mol) N-(2-Hydroxy-C12/14-alkyl)-ethylendiamin geschmolzen und bei 80° C mit 122,0 g (1,0 Mol) Benzoesäure versetzt. Unter Stickstoffbespülung wurde innerhalb 2,5 Stunden auf 220° C erhitzt. Dabei wurden beginnend bei 180° C, 19,3 g Wasser abdestilliert. Nun wurde das Erhitzen bei einem Druck von 250 mbar fortgesetzt. Nach 3 Stunden war eine Temperatur von 235° C erreicht, wobei noch weitere 15,7 g Destillat angefallen waren.

Imidazolin Nr. 13 (Tabelle 1)

In einer Rührapparatur entsprechend Herstellungsbeispiel für Imidazolin Nr. 2 wurden 245,3 g (1,0 Mol) N-(2-Hydroxy-C12/14-alkyl)-ethylendiamin geschmolzen und bei 70° C mit 73,0 g (0,5 Mol) Adipinsäure versetzt. Die Temperatur erhöhte sich auf 95° C. Innerhalb 2,5 Stunden wurde unter Stickstoffbespülung auf 200° C erhitzt. Dabei wurden 16,3 g Wasser abdestilliert. Das Erhitzen wurde nun bei einem Druck von 240 mbar fortgesetzt. Nach 3 Stunden betrug die Temperatur 220° C, wobei noch weitere 19,3 g Destillat erhalten wurden.

Acetat des Imidazolins Nr. 4

14,1 g (0,141 Mol) Essigsäure, 60 %ig und 224,9 g Wasser wurden in einer Rührapparatur vorgelegt. Dazu wurden langsam 50,0 g (0,129 Mol) des Imidazolins Nr. 4 (hergestellt entsprechend Imidazolin Nr. 2) zugetropft. Nach kurzer Rührzeit war eine klare, viskose Lösung entstanden.

Acetat des Imidazolins Nr. 8

Die Herstellung erfolgte analog zur Herstellung des Imidazolins-Nr.4-Acetats.

Die antimikrobielle Wirkung der vorstehenden Imidazolin-Acetate geht aus folgender Tabelle hervor:

| | Staphylococcus aureus SG 511 | Candida albicans ATCC 10 231 |
|---|---|---|
| Imidazolin-Nr.4-acetat | < 10 ppm | 50 ppm |
| Imidazolin-Nr.8-acetat | < 10 ppm | 50 ppm |

Die Prüfung erfolgte in Hemmreihen in flüssigem Nährsubstrat gegenüber den genannten Keimen im Röhrchentest CII1 gemäß Richtlinien der DGHM für chemische Desinfektionsmittel.

Phosphat des Imidazolins Nr. 8

In einer Rührapparatur wurden 10,8 g (0,094 Mol) Phosphorsäure, 85 %ig und 205,2 g Wasser vorgelegt. Dazu wurden 50,0 g (0,142 Mol) des in 30,0 g Isopropanol gelösten Imidazolins Nr. 8 (hergestellt entsprechend Imidazolin Nr. 2) getropft und so lange gerührt, bis eine klare Lösung entstanden war. Der pH-Wert betrug 6,32.

Die Korrosionsschutzwirkung des Imidazolin-Nr.8-Phosphats wurde im Korrosionstest in Anlehnung an DIN 51 360 an Graugußspänen geprüft. Dabei trat Rost lediglich in geringen Spuren auf. Der Test wird in folgender Weise durchgeführt:

Ein Schwarzbandfilter (Ø 9 cm) wird in eine Petrischale gelegt, mit 3 g Graugußspänen auf der ganzen Fläche überschichtet und anschließend mit 3 ml einer 3 %igen Lösung des Imidazolinphosphates angefeuchtet. Die Petrischale wird abgedeckt, 2 Stunden bei Raumtemperatur stehen gelassen, danach durch Waschen von den Spänen befreit und getrocknet. Die Auswertung erfolgte an Hand der Rostflecken auf dem Filtrierpapier im Vergleich zu einem mit Leitungswasser erhaltenen Blindwert.

Die Herstellung der in der nachfolgenden Tabelle 2 enthaltenen Imidazoliniumsalze der Formel II erfolgt durch Quaternierung von N-Hydroxyalkylimidazolinen der Tabelle 1 mit Methylchlorid in alkoholischer Lösung im Autoklaven (Substanzen Nr. 1 - 12 der Tabelle 2) beziehungsweise durch Quaternierung mit Benzylchlorid (Substanz Nr.6/1 der Tabelle 2).

/10

**Tabelle 2**

Imidazoliniumsalze

| Imidazolin aus Tabelle 1 Nr. | Konzentration in wäßriger Lösung % | Äquivalenzverhältnis $N_{KJ}$ | $Cl_{gs}$ | $Cl^{(-)}$ | Eptontit. | Imidazolingehalt (UV-Absorption bei 230 nm) |
|---|---|---|---|---|---|---|
| 1 | 20 | 2 | 1,17 | 0,96 | 0,95 | |
| 2 | 30 | 2 | 1,01 | 1,04 | 0,80 | 1,13 |
| 3 | 20 | 2 | 1,03 | 0,96 | 0,93 | |
| 4 | 20 | 2 | 0,92 | 0,85 | 0,85 | 0,96 |
| 5 | 30 | 2 | 1,06 | 1,06 | 0,85 | 0,92 |
| 6 | 30 | 2 | 0,97 | 0,99 | 0,97 | 1,03 |
| 7 | 10 Emulsion | 2 | 1,06 | 1,03 | 0,96 | 0,98 |
| 8 | 20 | 2 | 0,94 | 0,92 | 0,93 | 1,00 |
| 9 | 10 Emulsion | 2 | 1,18 | 1,09 | 0,98 | 1,10 |
| 12 | 30 | 2 | 0,94 | 0,95 | 0,93 | + |
| 6/1 | 30 | 2 | 0,95 | 0,92 | 0,92 | + |

Erläuterungen: $N_{KJ}$ = Stickstoff nach Kjeldahl
$Cl_{gs}$ = Gesamt-Chlorgehalt
$Cl^{(-)}$ = ionogenes Chlor

Eptontit. = Titration nach Epton
+ = spektroskopisch nicht zu ermitteln

/11

Beispiele für die Herstellung von Imidazoliniumsalzen der Formel (II):

<u>Imidazoliniumsalz Nr. 3 der Tabelle 2</u>
80,0 g (0,221 Mol) des N-Hydroxyalkylimidazolins Nr. 3 der Tabelle 1 wurden in 80 g Methanol gelöst und in einem Autoklaven mit 20 g (0,395 Mol) Methylchlorid unter 10 bar Stickstoffdruck umgesetzt. Nach einer Reaktionszeit von 3 Stunden bei 75° C wurde abgekühlt und der Autoklav entspannt. Anschließend wurden das Methanol sowie überschüssiges Methylchlorid abdestilliert und der Rückstand mit Wasser zu einer 30 %igen Lösung verdünnt.

<u>Imidazoliniumsalz Nr. 9 der Tabelle 2</u>
Eine Lösung aus 70,0 g (0,12 Mol) des N-Hydroxyalkylimidazolins Nr. 9 der Tabelle 1 und 70,0 g Methanol wurden in einem Autoklaven vorgelegt. Nach dem Verschließen der Apparatur wurden 9,1 g (0,18 Mol) Methylchlorid aufgedrückt und 4 Stunden bei 75° C zur Reaktion gebracht. Anschließend wurde abgekühlt, entspannt und das Methanol sowie überschüssiges Methylchlorid abdestilliert. Das Endprodukt wurde mit Wasser zu einer 10 %igen, gelartigen Emulsion verdünnt.

<u>Imidazoliniumsalz Nr.6/1 der Tabelle 2</u>
55,0 g (0,26 Mol) des Imidazolins Nr. 6 der Tabelle 1 wurden in 30 ml Isopropanol gelöst und mit 32,6 g (0,26 Mol) Benzylchlorid in 3 Stunden bei 80 - 85° C quaterniert. Danach wurde das Isopropanol abdestilliert und das Produkt mit Wasser auf 30 % Aktivsubstanz eingestellt.

Die Ergebnisse der Prüfung der mikrobiziden Wirkung einiger Imidazoliniumsalze der Tabelle 2 finden sich in der nachfolgenden Tabelle 3:

## Tabelle 3

Mikrobizide Wirkung von Imidazoliniumsalzen der Tabelle 2 im Suspensionstest

| Imidazoliniumsalz Nr. der Tabelle 2 | ppm | Keimzahl / ml in der Impfsuspension: $3,0 \times 10^8$ Staphylococcus aureus | $3,7 \times 10^8$ Escherichia coli | $1,6 \times 10^8$ Pseudomonas aeruginosa | $1,9 \times 10^8$ Candida albicans | pH |
|---|---|---|---|---|---|---|
| 8 | 50 | 5 | 10 | 10 | 40 | 5,2 |
| | 10 | 5 | 60 | 40 | 120 | |
| 6 | 100 | ≤ 2,5 | ≤ 2,5 | 10 | 40 | 5,8 |
| | 50 | 20 | 60 | 120 | x | |
| | 10 | 40 | x | x | x | |
| 4 | 50 | 5 | 10 | 10 | 20 | 5,4 |
| | 10 | 5 | 60 | 10 | 20 | |
| 10 | 100 | | | ≤ 2,5 | 40 | 6,0 |
| | 50 | ≤ 2,5 | ≤ 2,5 | 10 | x | |
| | 10 | 5 | 10 | 10 | x | |
| 3 | 50 | ≤ 2,5 | ≤ 2,5 | 10 | 5 | 6,1 |
| | 10 | ≤ 2,5 | 5 | 60 | 20 | |
| 1 | 100 | ≤ 2,5 | ≤ 2,5 | 10 | 10 | 5,5 |
| | 50 | ≤ 2,5 | 20 | 20 | 40 | |
| | 10 | 60 | x | | | |

Wirkstoffe gelöst in : $H_2O$

Geprüfte Zeiten: 2,5-5-10-20-40-60-120 Minuten

Konzentration auf Wirkstoff bezogen

Temperatur: Raumtemperatur

Enthemmungsmittel: Tween-Lecithin

Impfsuspension 1:100 in Wirkstoffansatz verdünnt

Zahlen: Abtötungszeit in Minuten

x = > 120 Minuten



BAD ORIGINAL

Imidazoliniumsalz Nr. 8 der Tabelle 2

Das Imidazoliniumsalz Nr. 8 wurde in analoger Weise wie das Imidazoliniumsalz Nr. 3 hergestellt.

Zur Prüfung auf Avivagewirkung wurden bei einem Flottenverhältnis von 1 : 20 0,25 % Aktivsubstanz, bezogen auf das Warengewicht, im Ausziehverfahren auf ein Frotteegewebe aus Zellulosematerial appliziert. Das behandelte Gewebe wurde anschließend kalt gespült, trockengeschleudert, an der Luft getrocknet und hinsichtlich des Avivageeffektes und der Wiederbenetzbarkeit geprüft. Im Vergleich zu einem herkömmlichen Quartärammoniumsalz (Distearyldimethylammoniumchlorid) wurde bei gleich gutem Avivageeffekt eine wesentlich bessere Wiederbenetzbarkeit festgestellt.

/14

BAD ORIGINAL

Neue N-Hydroxyalkylimidazolinderivate, deren Herstellung und Verwendung

---

Patentansprüche

1. Neue N-Hydroxyalkylimidazolinderivate der Formel

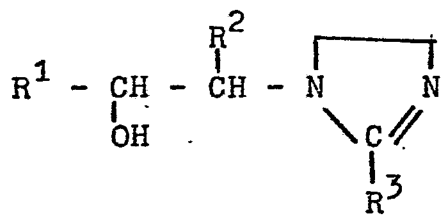 (I)

oder

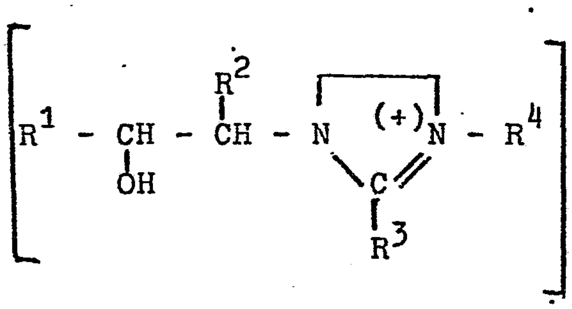 $X^{(-)}$ (II)

worin bedeuten:

$R^1$, $R^2$ = H und/oder unverzweigter $C_1$-$C_{2o}$-Alkylrest, wobei die Summe der C-Atome in $R^1$ und $R^2$ 8 - 2o ist,

$R^3$ = H oder ein $C_1$-$C_{23}$-Alkylrest oder ein Phenylrest oder ein Rest $R^5$ - A, wobei $R^5$ ein um die Carboxylgruppen verminderter Rest einer aliphatischen oder aromatischen $C_3$-$C_{12}$-Dicarbonsäure, A ein um $R^3$ verminderter Hydroxyalkylimidazolinrest ist,

$R^4$ = $C_1$-$C_{2o}$-Alkyl oder Benzyl oder Propylsulfonat,

$X^{(-)}$ = beliebiges Anion einer organischen oder anorganischen Säure, insbesondere $Cl^{(-)}$, $X^{(-)}$ entfällt, wenn $R^4$ = Propylsulfonat.

2. Verfahren zur Herstellung von N-Hydroxyalkylimidazolinen der Formel (I) des Anspruchs 1, dadurch gekennzeichnet, daß man ein N-Hydroxyalkylethylendiamin der Formel

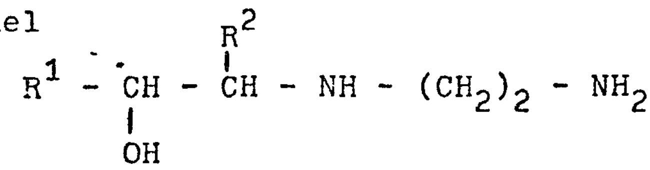

(III)

worin $R^1$, $R^2$ die im Patentanspruch 1 angegebene Bedeutung haben,

mit einer annähernd äquivalenten Menge einer Mono- oder Dicarbonsäure der Formel

$$R^3\text{-COOH oder HOOC-}R^5\text{-COOH,}$$

worin $R^3$ und $R^5$ die im Patentanspruch 1 angegebene Bedeutung haben, bei 12o - 25o° C unter Abdestillieren von 2 Mol Reaktionswasser pro Imidazolinring-Äquivalent umsetzt.

3. Verfahren zur Herstellung von Imidazoliniumsalzen der Formel (II) des Anspruchs 1, dadurch gekennzeichnet, daß man ein N-Hydroxyalkylimidazolin der Formel (I) des Anspruchs 1 mit einem Quaternierungsmittel bei 6o - 1oo° C umsetzt.

4. Verfahren nach Anspruch 3, dadurch gekennzeichnet, daß man als Quaternierungsmittel Methylchlorid, Benzylchlorid, Dimethylsulfat, Trimethylphosphat oder Propansulton verwendet.

5. Verwendung der N-Hydroxyalkylimidazoline der Formel (I) des Anspruchs 1 als Korrosionsschutzmittel oder mikrobizide Mittel oder als Zwischenprodukte zur Herstellung der Imidazoliniumsalze der Formel (II) des Anspruchs 1.

6. Verwendung der Imidazoliniumsalze der Formel (II) des Anspruchs 1 als mikrobizide Mittel oder als Körper- und Haarwaschmittel oder als Textilavivage- und -weichmachungsmittel.

7. Verwendung der Imidazoliniumsalze der Formel (II) des Anspruchs 1 als Weichmachungsmittel für Synthese- oder Zellulosematerial oder Mischungen derselben.

0023335

Europäisches Patentamt

**EUROPÄISCHER RECHERCHENBERICHT**

Nummer der Anmeldung

EP 80 10 4280

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 2 968 623 (DARLING)<br>* Spalte 5 *<br>-- | 1,2 |
| X | GB - A - 776 487 (WILSON)<br>* Seite 5, Zeilen 116-125; Figur 29 *<br>-- | 1,2 |
| A | FR - A - 2 308 625 (HOECHST)<br>* Seiten 2-5 *<br>---- | 1-4,6 |

**KLASSIFIKATION DER ANMELDUNG (Int Cl.³)**

C 07 D 233/14
233/08
C 23 F 11/14
11/16
A 01 N 43/50
D 06 M 13/46
C 11 D 1/62
A 61 K 7/08

**RECHERCHIERTE SACHGEBIETE (Int. Cl.³)**

C 07 D 233/14
233/08
C 23 F 11/14
11/16
A 01 N 43/50
D 06 M 13/46
C 11 D 1/62
A 61 K 7/08

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 28-10-1980 | DE BUYSER |

EPA form 1503.1 06.78